# NEW EUROPEAN PATENT SPECIFICATION

(11) **EP 3 372 093 B2**
(45) Date of publication and mention of the opposition decision: **14.12.2022**
(45) Mention of the grant of the patent: 31.07.2019
(21) Application number: 17203958.8
(22) Date of filing: 17.01.2013
(51) Int. Cl.: A23L 19/00, A61K 8/97, A61Q 5/00, C11D 3/00, A23K 10/10, A23L 33/21

(54) **PROCESS FOR OBTAINING CITRUS FIBER FROM CITRUS PEEL**
VERFAHREN ZUR GEWINNUNG VON ZITRUSFASERN AUS ZITRUSSCHALE
PROCÉDÉ POUR L'OBTENTION DE FIBRES D'AGRUMES À PARTIR D'ÉCORCES D'AGRUMES

(30) Priority: 20.01.2012 US 201261588915 P
(43) Date of publication of application: 12.09.2018
(62) Divisional of application: 13703924.4
(73) Proprietor: Cargill, Incorporated, Wayzata, MN 55391 (US)
(72) Inventor: GUSEK, Todd Walter, Crystal, 55422 (US); MAZOYER, Jacques Andre Christian, F-50500 Carentan (FR); REEDER, David Hiram, Chanhassen, Minnesota 55317 (US); WALLECAN, Joel Pierre Rene, B-1800 Vilvoorde (BE)
(74) Representative: Forresters IP LLP

(56) References cited:
- EP-A1- 0 485 030
- WO-A1-2011/131457
- WO-A1-2012/016190
- WO-A2-2012/016201
- HUANG D ET AL: "Production of water-soluble orange peel fiber used as dietary food material involves dipping fresh orange peel in Vitamin C or citric acid solution, drying, crushing, extracting, centrifuging extract, adding water, and pulverizing", WPI / THOMSON,, vol. 2010, no. 62, 11 August 2010 (2010-08-11), XP002710484,

## Description

### CROSS REFERENCE TO RELATED APPLICATION

This application claims the benefit of the United States Provisional Patent Application, Serial No. 61/588,915, filed January 20, 2012, PROCESS FOR OBTAINING CITRUS FIBER FROM CITRUS PEEL.

### TECHNICAL FIELD

The present invention is directed to a process for obtaining citrus fiber from citrus peel. The resulting dried citrus fiber is useful as a food additive in food products, feed products and beverages. The citrus fiber is also useful in personal care, pharmaceutical or detergent products.

### BACKGROUND OF THE INVENTION

Citrus peel is a waste product from the juice industry or is a by-product from the pectin process. Pectin can be obtained from various raw materials like citrus peels using a specific treatment in hot acid aqueous conditions. Citrus include e.g. lemon, orange, lime, grape fruit of which lemon is the most commonly used. The peels obtained from the juice industry are typically subjected to another treatment to extract juice and oil, and are then dried. These peels are typically used for cattle feed. The peels obtained from the pectin process, after extraction of pectin, are of even lower economical value. Huang et al (2010), CN-A-101797037, disclose the production of orange peel fiber. WO-A-2012/016201 discloses a process for modifying the properties of citrus fiber. WO-A-2012/016190 discloses a process for obtaining citrus fiber from citrus pulp. EP-A-0 485 030 discloses fruit juice citrus fiber from pulp. WO-A- 2011/131457 discloses a beverage comprising soy protein and citrus fibers.

It would thus be desirable to improve the economical value of this product. One approach is to improve the functional properties of the fibers which can be derived from citrus peel. However, the properties of existing citrus peel fibers can still be improved.

Hence, it is an object of the present invention to develop a process for obtaining citrus fiber from citrus spent peel having improved properties versus the citrus fiber of the prior art. It is further an object of the present invention to provide a method to obtain a citrus fiber (not according to the present invention) which has good hydration ability and viscosifying properties.

### SUMMARY OF THE INVENTION

The present invention is defined by the appended claims. The present invention, according to one aspect, is directed to a process for obtaining citrus fiber from citrus peel. Citrus peel is treated to obtain homogenized citrus peel. The process further comprises a step of washing the homogenized citrus peel with an organic solvent to obtain organic solvent washed citrus peel. The organic solvent washed citrus peel is desolventized and dried, and citrus fiber is recovered.

### BRIEF DESCRIPTION OF THE DRAWINGS

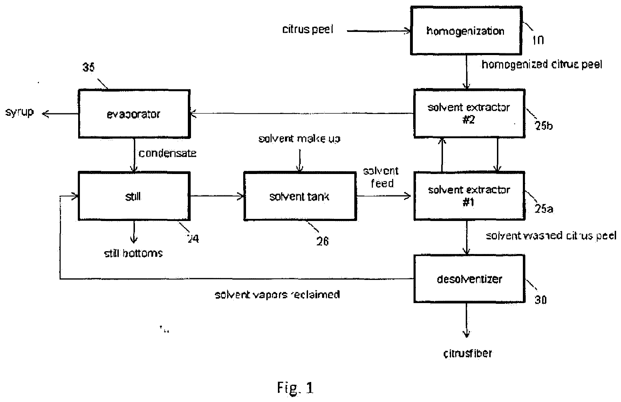

FIG. 1 is a schematic illustration of a process in accordance with a preferred embodiment of the present invention.

### DETAILED DESCRIPTION OF THE INVENTION

In one aspect, the present invention is directed to a process for obtaining citrus fiber from spent citrus peel.

The process according to the present invention may be used for obtaining citrus fiber from citrus peel from a wide variety of citrus fruit, non-limiting examples of which include oranges, tangerines, limes, lemons, and grapefruit. In one preferred embodiment, citrus fiber is obtained from lemon peel.

In the process according to the present invention, spent citrus peel is treated to obtain homogenized citrus peel. In the invention, spent peel is used as starting material. Optionally, the citrus peel may be washed with water prior to homogenization. Sometimes, citrus peel may be provided in a frozen or dried state which requires thawing or rehydration prior to homogenization. Preferably, the citrus peel is adjusted with water to a dry matter content of 5 wt% or less. While not intending to be bound to any theory, it is believed that the homogenization treatment causes disruption and disintegration of whole peel cells and cell fragments. Homogenization is effected by pressure homogenization. Preferably, the power input (power per unit volume) for effecting homogenization is at least about 1000 kW per cm³ of citrus peel.

In the present invention, the homogenization treatment is a pressure homogenization treatment. Pressure homogenizers typically comprise a reciprocating plunger or piston-type pump together with a homogenizing valve assembly affixed to the discharge end of the homogenizer. Suitable high pressure homogenizers include high pressure homogenizers manufactured by GEA Niro Soavi, of Parma (Italy), such as the NS Series, or the homogenizers of the Gaulin and Rannie series manufactured by APV Corporation of Everett, Massachusetts (US).

During the pressure homogenization, the citrus peel is subjected to high shear rates as the result of cavitations and turbulence effects. These effects are created by the citrus peel entering the homogenizing valve assembly from the pump section of the homogenizer at a high pressure (and low velocity). The process of the present invention requires pressures of from about 50 bar to about 1000 bar.

Depending on the particular pressure selected for the pressure homogenization, and the flow rate of the citrus peel through the homogenizer, the citrus peel may be homogenized by one pass through the homogenizer. However, more than one pass of the citrus peel may be required.

It is disclosed that the citrus peel is homogenized by a single pass through the homogenizer. In a single pass homogenization, the pressure used is preferably from about 300 bar to about 1000 bar, more preferably from about 400 bar to about 800 bar, even more preferably from about 500 bar to about 750 bar.

It is disclosed that the citrus peel is homogenized by multiple passes through the homogenizer, preferably at least 2 passes, more preferably at least 3 passes through the homogenizer. In a multipass homogenization, the pressure used is typically lower compared to a single-pass homogenization and preferably from about 100 bar to about 600 bar, more preferably from about 200 bar to about 500 bar, even more preferably from about 300 bar to about 400 bar.

Optionally, the citrus peel may be subjected to a heat treatment prior to homogenization. Preferably, the temperature used in the heat treatment can vary from about 50°C to about 140°C for a period of from about 1 second to about 3 minutes. The heat treatment may be used for pasteurization of the citrus peel. For pasteurization, the heat treatment preferably employs a temperature of from about 65°C to about 140°C, preferably from about 80°C to about 100°C for a period of from about 2 s conds to about 60 seconds, preferably from about 20 seconds to about 45 seconds. Pasteurization is preferred to inactivate pectinesterases for preventing cloud loss and to inactivate spoilage microorganisms for enhancing storage stability.

The homogenized citrus peel is then contacted with an organic solvent. In one aspect, the organic solvent extracts water, flavors, odors, colors and the like from the citrus peel. The solvent should preferably be polar and water-miscible to better facilitate removal of the desired components. Available solvents may include lower alcohols such as methanol, ethanol, propanol, isopropanol, or butanol. Preferred solvents are ethanol, isopropanol, and combinations thereof. The solvent may be provided in aqueous solution. The concentration of solvent in the solvent solution most often ranges from about 70 wt% to about 100 wt%. In one embodiment, a 75 wt% aqueous ethanol solution is used as solvent. In a preferred embodiment, a 90 wt% aqueous ethanol solution is used as solvent. In yet another preferred embodiment, isopropanol is used as a solvent, preferably an aqueous isopropanol solution. In general, solvents will remove water-soluble components at lower concentrations and oil-soluble components at higher concentrations. Optionally, a more non-polar co- solvent may be added to the aqueous alcohol to improve the recovery of oil-soluble components in the citrus peel. Examples of such non-polar solvents include ethyl acetate, methyl ethyl ketone, acetone, hexane, methyl isobutyl ketone and toluene. The more non-polar solvents may be added at up to about 20% of the solvent mixture. Many solvents, such as ethanol, have a lower heat of vaporization than that of water, and therefore require less energy to volatilize than would be needed to volatilize an equivalent mass of water. The solvent preferably is removed and reclaimed for reuse.

Preferably, the citrus peel is contacted with organic solvent at a solids-to-solvent weight ratio of at least about 0.25:1, preferably at least about 0.5:1, and often at least about 0.75:1, from about 1 :1 to about 5:1, or from about 1.5:1 to about 3:1, based on the wet weight of the solids. In one embodiment, the solids-to-solvent ratio is about 2:1.

Extraction can be accomplished using a single stage but preferably is performed using multistage extraction, e.g., a two-, three-, or four-staged extraction process, and preferably using countercurrent extraction. There is no particular upper limit contemplated on the number of extraction stages that may be used. Fig. 1 schematically illustrates a preferred embodiment in which a two-stage countercurrent extraction process employs first and second solvent extractors **25a** and **25b,** respectively.

After homogenization **10,** homogenized citrus peel is fed into the second extractor **25b.** An aqueous ethanol or isopropanol solvent is fed from a solvent tank **26** into the first solvent extractor **25a.** Spent solvent from the first solvent extractor **25a** is fed into the second solvent extractor **25b,** while the extracted citrus peel from the second solvent extractor **25b** are fed into the first solvent extractor **25a.** Spent solvent from the second solvent extractor **25b** may be fed into an evaporator **35** (optional) to separate solids (e.g., sugars, acids, colors, flavors, citrus oils, etc.) from the spent solvent, which can be condensed and returned to a still **24.** Still bottoms (predominately water) are separated and removed.

After each extraction stage, liquid is preferably further removed. One suitable device is a decanter centrifuge. Alternatively, a sieve, a belt filter press or other device suitable for removing liquids, may be used.

Citrus peel from the first solvent extractor **25a** is fed to a desolventizer **30.** The desolventizer **30** removes solvent and water from the solids remaining after extraction, enabling the solvent to be reclaimed for future use and also ensuring that the product is safe for milling and commercial use. The desolventizer **30** can employ indirect heat to remove significant amounts of solvent from the solid residue. Alternatively, direct heat can be provided for drying, e.g., by providing hot air from flash dryers or fluidized bed dryers. Direct steam may be employed, if desired, to remove any trace amounts of solvent remaining in the solids. Vapors from the desolventizer **30** preferably are recovered and fed to the still **24** to reclaim at least a portion of the solvent.

Retention time in each extraction step may vary over a wide range but can be about 5 minutes or less per extraction step. The temperature in the solvent extractors) depends on such factors as the type of solvent used but most often ranges from about 4°C to about 85°C at atmospheric pressure. Temperatures can be appropriately increased or decreased for operation under super- or subatmospheric pressures. Optionally, techniques such as ultra-sound are used for enhancing efficiency of the extraction process. By maintaining a closed system, solvent losses during extraction, desolventizing, and distillation can be minimized. Preferably, at least about 70 wt% of the solvent is recovered and reused. A solvent make-up stream delivers fresh solvent into the solvent tank 26 to replenish any solvent that is not recovered.

It is disclosed that the process further comprises a comminuting or pulverizing step prior to desolventizing and drying. Suitable methods include, but are not limited to, grinding, milling, crushing, high speed mixing, or impingement. Comminution or pulverization can be beneficial to disintegrate any clumps or aggregates that are left after the removal of liquid with the belt filter pressing step. This step furthermore facilitates the removal of solvent. While not wishing to be bound by theory, it is believed that comminution or pulverization further opens the fibers. As a result of this, the solvent is more uniformly distributed and easier to be removed in the subsequent desolventization and drying step. In yet another preferred embodiment, the comminuting or pulverizing step is used in combination with adding and dispersing water or a blend of water and an organic solvent (as described hereinbefore) to enhance desolventization and drying, and achieve the desired humidity in the finally obtained citrus fiber for a particular end use.

In another preferred embodiment, the process according to the present invention further comprises a comminuting or pulverizing step after drying. This post-drying comminuting or pulverizing step may be carried out to further reduce the particle size of the citrus fiber, to improve flowability, dispersability, and/or hydration properties.

In yet another preferred embodiment, the process according to the present invention further comprises the step of subjecting the citrus peel to a processing aid. Preferably, the processing aid is selected from the group consisting of enzymes, acids, bases, salts hydrocolloids, vegetable fiber, bleaching agent, and combinations thereof. In one embodiment, the processing aid is mixed with the citrus peel prior to homogenization. In another embodiment, the processing aid is added after homogenization.

In one aspect the processing aid may be used to tailor the properties of the finally obtained citrus fiber.

Preferred enzymes include, but are not limited thereto, pectinase, protease, cellulase, hemicellulase and mixtures thereof. When enzymes are used, they are to be used prior to any heat treatment that would inactivate them, and preferably also prior to homogenization. Inactivation by heat treatment is however desired once the desired effect is achieved.

Preferred acids include, but are not limited thereto, citric acid, nitric acid, oxalic acid, ethyl enediaminetetraacetic acid and combinations thereof. Citric acid is however most preferred as it is a food grade acid.

A preferred base is caustic soda. Caustic soda improves the swelling behavior and the texurising properties of the citrus fiber. Caustic soda is preferably added after homogenization. A preferred salt is sodium chloride.

Preferred hydrocolloids include, but are not limited thereto, pectin, alginate, locust bean gum, xanthan gum, guar gum, carboxymethylcellulose and combinations thereof.

A bleaching agent may further enhance the color properties (i.e. render the citrus fiber whiter). A preferred bleaching agent is hydrogen peroxide.

The citrus fiber (not according to the present invention) obtained by the process according to the present invention has improved properties over other citrus fibers from the prior art. Especially, the citrus fiber has good swelling behavior, hydration ability and viscosifying properties. It is capable of building viscosity under relatively low shear in aqueous media.

The citrus fiber (not part of the claimed invention) has a c* close packing concentration of less than 3.8 wt%, anhydrous basis. Preferably, the c* close packing concentration is less than 3.6, even more preferably less than 3.4wt%, and most preferably less than 3.2 wt%, anhydrous basis. The determination of the c* close packing concentration is described in the test method section herein below.

The citrus fiber (not part of the claimed invention) preferably has a moisture content of about 5% to about 15%, more preferably from about 6% to about 14%. Preferably, at least about 90% of the volume of the particles have a diameter of less than about 1000 micrometers, preferably from about 50 micrometers to about 1000 micrometers, more preferably from about 50 micrometers to about 500 micrometers, even more preferably from about 50 micrometers to about 250 micrometers.

The citrus fiber (not part of the claimed invention) preferably has a viscosity of at least about 1000 mPa.s, wherein said citrus fiber is dispersed in standardized water at a mixing speed of from 800 rpm to 1000 rpm, preferably 900 rpm, to a 3 w/w% citrus fiber/standardized water solution, and wherein said viscosity is measured at a shear rate of 5 s⁻¹ at 20°C. Preferably, the viscosity at a shear rate of 5 s⁻¹ at 20°C is at least about 2000 mPa.s, more preferably at least about 3000 mPa.s, even more preferably at least 4000 mPa.s, even more preferably at least 5000 mPa.s and up to 15000 mPa.s. The preparation of the standardized water, and the method for measuring viscosity is described in the test method section herein below.

The citrus fiber (not according to the present invention) further has good emulsification properties.

The citrus fiber (not according to the present invention) also has excellent whiteness properties. The citrus fiber (not according to the present invention) may have a CIELAB L* value of at least about 85, preferably of at least about 90, more preferably at least about 92, even more preferably at least about 93. Preferably, the citrus fiber (not according to the present invention) has a CIELAB b* value of less than about 20, even more preferably of less than about 15. The method for determining the CIELAB L* and b* values is described in the test method section herein below.

The citrus fiber (not according to the present invention) can be blended with other fibers, such as plant-derived (e.g. from vegetables, grains/cereals) fibers, with other citrus fibers such as citrus fiber obtained from citrus pulp, or combinations thereof. The blend can be in dry or liquid form.

The citrus fiber (not according to the present invention) and the blends described hereinbefore may be used in food applications, feed applications, beverages, personal care products, pharmaceutical products or detergent products. The amount of citrus fiber (not according the present invention) (or blend) to be used depends on the given application and the desired benefit to be obtained, and lies within the knowledge of a skilled person.

Food applications may include, but are not limited to, dairy products, frozen products, bakery products, fats and oils, fruit products, confectionary, meat products, soups, sauces and dressings. Dairy products include, but are not limited to yoghurt, fromage frais, quark, processed cheese, dairy desserts, mousses. Frozen products include, but are not limited to, ice cream, sorbet, water ice. Bakery products include, but are not limited to, cakes, sweet goods, pastry, patisserie, extruded snacks, fried snacks. Fats and oils include, but are not limited to, margarines, low fat spreads, cooking fats. Fruit products include, but are not limited to, fruit preparations, yoghurt fruit preparations, conserves, jams, jellies. Sauces and dressings include, but are not limited to, egg yolk or egg-yolk derivatives containing oil/water emulsions such as mayonnaise, and ketchup. Confectionary includes, but is not limited to, candy, chocolate spreads, nut-based spreads. Meat products include, but are not limited to, chilled or frozen processed meat and poultry, preserved meat products, fresh sausage, cured sausage and salami.

Beverages may include concentrates, gels, energy drinks, carbonated beverages, non-carbonated beverages, syrups. The beverage can be any medical syrup or any drinkable solution including iced tea, and fruit juices, vegetable based juices, lemonades, cordials, nut based drinks, cocoa based drinks, dairy products such as milk, whey, yogurts, buttermilk and drinks based on them. Beverage concentrate refers to a concentrate that is in liquid form.

Personal care products may include cosmetic formulations, hair care products such as shampoos, conditioners, creams, styling gels, personal washing compositions and sun creams.

Detergent products may include hard surface cleaning products, fabric cleaning or conditioning products.

### Test methods

### 1. Preparation of Standardised water

Dissolve 10.0g NaCl (e.g. Merck 1.06404.1000, CAS [7647-14-5]) and 1.55g CaCl₂.2H₂O (e.g. Merck 1.02382.1000, CAS [10035-04-8]) in low conductivity water (e.g. milli-Q Ultrapure Millipore 18.2 MΩcm), and make up to 1 liter to prepare standardized water stock. Take a 100ml aliquot of the standardized water stock and make up to 1 liter with low conductivity water.

### 2. Measuring c* close packing concentration

### 2.1 Principle

Citrus fiber samples (n ≥ 10) are wetted with ethylene glycol, dispersed in standardised tap water, and subjected to the MCR301 controlled shear stress (CSS) oscillatory test. The citrus fiber dispersions are measured by 0.25w/w% increments in the range of 1.75-5.00w/w%. The linear viscoelastic range (LVR) complex moduli G* is plotted against concentration. The close-packing concentration c* is determined via the two tangents crossover method on a linear scale.

### 2.2 Apparatus

- Anton Paar MCR301 rheometer with coaxial cylinder configuration (TEZ150P-CF Peltier at 20°C) with vane probe ST24-2D/2V/2V-3D, grooved measuring cup CC27/T200/SS/P and circulating cooling water bath set at 15 °C. The equipment must be clean and dry, and the MCR301 units must be turned on 30 minutes before use. Checks should be made according to the instruction manual of the supplier (see instruction manual). The Circulator bath and pump should be at all times in use to avoid burning of the peltier unit. According to the manufacturer, the water bath must be filled with demineralised water containing maximum 30 % of antifreeze (e.g. ethylene glycol).
- RWD 20 Digital IKA stirrer and lower the paddle (4 bladed propeller 07 410 00)
- 600 ml Duran glass beaker (ø 10cm)
- Laboratory balance having a precision of 0.01 g
- Hard plastic soup spoon

### 2.3 Procedure

### System Start-up

Start up the circulator bath (filled with demineralised water + 30% ethylene glycol (e.g. Merck 1.00949.1000, CAS [107-21-1])) and afterwards the rheometer according to the procedure explained in the instruction manual. Select the workbook and perform the initialisation procedure according to the instruction manual.

### System calibration

The standard calibration check procedure for the MCR301 is fully described in the instruction manual and should be performed according to the instruction manual. The MCR301 instruments must be ready (initiated and all parameters checked) before testing the citrus fiber dispersions. The ST24 measuring system CSR should be set to 1 and the CSS value (Pa/mNm) should be fixed with certified calibration Newtonian oil (e.g. Cannon N100, available from Cannon Instrument Company, State College, PA 16803, USA).

### Sample preparation

- Place a 600 ml glass beaker on the laboratory balance, and zero the balance.
- Weigh into the beaker the required grams (x) of citrus fiber, to the nearest 0.01 g, according to the moisture content (m) of the citrus fiber sample: x=3c/[(100-m)/100], for any given concentration c in w/w% (samples starting at 1.75 w/w%, to 5.00 w/w% with 0.25w/w% increments). The moisture content m should be determined by infra-red moisture balance (Sartorius MA 30), as weight loss at 105°C with automatic timing, typically 3-4g citrus fiber covering the entire bottom of the aluminiun pan. The moisture content (m) of citrus fiber is in weight percent (w%).
- Weigh into a second 600mL beaker the required grams (w) of standardised tap water, to the nearest 0.1 g, according to the moisture of the citrus fiber sample: w=270.0-x
- Place the beaker with CPF on the laboratory balance, zero the balance, add 30.0g (to the nearest 0.1 g) of ethylene glycol, put the beaker out of the balance and mix the content with the plastic spoon thereby wetting the whole powder (this operation is performed within 60 seconds).
- Pour at once the standardised tap water on to the wet citrus fiber and mix the content with the plastic spoon by repeated clockwise and anti-clockwise rotations (this operation is performed within 60 seconds).
- Position the glass beaker with its content (citrus fiber, ethylene glycol, standardised tap water) underneath a RWD 20 Digital IKA stirrer and lower the paddle (4 bladed propeller 07 410 00) into the paste until 2cm from the bottom of the glass beaker.
- Adjust the paddle speed (rpm) to 900 rpm and stir 10 minutes at 900 rpm.
- Cover the beaker with aluminum foil and allow 24 hours rest prior measurement
- Pour the required amount of CPF dispersion into the cylindrical cup of the rheometer and insert immediately the vane probe ST24 (starch cell probe) into the cylinder containing the CPF dispersion

### Sample analysis

- Perform CSS oscillatory test with the MCR301 according to the manual instructions, with 2 segments:
   segment 1: non recording, 10 minutes at 20°C (equilibration)
   segment 2: recording, 1971 seconds at 20°C, 50 measuring points integration time 100 to 10 seconds log, torque 1 to 10,000µNm log, frequency 1Hz

### Results

At low stress, where the G* (*versus* stress) is showing constant plateau values, average the G* results over the linear viscoelastic range. Using the software "LVE Range", the end of the linear viscoelastic region in the CSS experiments can be determined.

Plot the LVR G* *versus* concentration. The first tangent at low concentration (below c*) has a much lower slope than the second tangent at high concentration (above c*). Using linear fitting (e.g. with Microsoft^{®} Excel^{®}), the crossover point of both tangents occurs at the c* close packing concentration.

### 3. Measuring Viscosity

Add citrus fiber to standardized water in a beaker with a paddle mixer to obtain a 3 wt% citrus fiber dispersion with a total volume of 300 ml. Prior to adding the citrus fiber, create a vortex by adjusting the paddle speed to 900 rpm using an IKA Overhead Mechanical Stirrer RW20 equipped with a 4-bladed propeller stirrer. Then add the citrus fiber quickly (before the viscosity builds up) on the walls of the vortex under stirring (900 rpm using an IKA Overhead Mechanical Stirrer RW20 equipped with a 4-bladed propeller stirrer). Continue stirring for 15 minutes at 900 rpm. Store the sample for 12 hours at 20°C.

Then perform the viscosity test with a rheometer (e.g. Anton Paar MCR300), in accordance with the rheometer's instructions, in function of shear rate (from 0.01 to 100 s⁻¹) at 20°C.

The viscosity (mPa.s) is determined at a shear rate of 5 s⁻¹.

### 4. Measuring Colour (CIELAB L*, b* values)

CIE *L*a*b**(CIELAB) is the most complete color space specified by the International Commission on Illumination (*Commission Internationale d'Eclairage*). It describes all the colors visible to the human eye and was created to serve as a device independent model to be used as a reference. The L*and b* values of the citrus fiber are obtained by placing citrus fiber (in powder form) in the glass cell (fill the cell to about a half) of the colorimeter and analyse the sample in accordance with the user's instructions of the colorimeter. The colorimeter used is a Minolta CR400 Colorimeter.

### Example

| sample | c* (wt%, anhydrous base) |
|---|---|
| Disclosed Citrus fiber (not according to the claimed invention) | 3.5 |
| Herbacel AQ-Plus Citrus Fibre N | 3.94 |
| Citri-Fi 100 | 4.04 |

| | |
|---|---|
| Citri-Fi 100: orange fiber derived from orange pulp (Fiberstar Inc.) Herbacel AQ-Plus Citrus Fibre N: lemon fibers derived from lemon peel (Herbstreith & Fox Inc). | |

## Claims

1. A process for preparing citrus fibers from spent citrus peel, the process comprising
a. treating spent citrus peel to obtain homogenized spent citrus peel;
b. washing the homogenized spent citrus peel with an organic solvent to obtain organic solvent washed spent citrus peel;
c. desolventizing and drying the organic solvent washed spent citrus peel; and
d. recovering citrus fiber therefrom; and
wherein the treatment comprises pressure homogenization using a pressure of from 50 bar to 1000 bar.

2. A process according to claims 1, wherein the citrus peel is subjected to a heat treatment prior to the homogenization treatment at a temperature of from 50 °C to 140°C for a period of 1 second to 3 minutes.

3. A process according to claims 1-2, wherein said process further comprises a comminution or pulverizing step prior to desolventizing and drying.

4. A process according to claims 1-3, wherein said process further comprises a comminution or pulverizing step after drying.

5. A process according to claims 1-4, wherein said process further comprises subjecting said citrus peel to a processing aid selected from the group consisting of enzymes, acids, bases, hydrocolloids, vegetable fiber, bleaching agents, and combinations thereof.

6. A process according to claims 1-5, wherein said process further comprises adding a processing aid selected from the group consisting of enzymes, acids, bases, hydrocolloids, vegetable fiber, bleaching agents, and combinations thereof.

## Patentansprüche

1. Verfahren zur Herstellung von Zitrusfasern aus verbrauchter Zitrusschale, wobei das Verfahren umfasst
a. Behandeln von verbrauchter Zitrusschale, um homogenisierte verbrauchte Zitrusschale zu gewinnen;
b. Waschen der homogenisierten verbrauchten Zitrusschale mit einem organischen Lösungsmittel, um mit organischem Lösungsmittel gewaschene verbrauchte Zitrusschale zu gewinnen;
c. Desolventisieren und Trocknen der mit organischem Lösungsmittel gewaschenen verbrauchten Zitrusschale; und
d. Wiedergewinnen von Zitrusfaser daraus;
und
wobei die Behandlung Druckhomogenisierung unter Verwendung eines Drucks von 50 bar bis 1000 bar umfasst.

2. Verfahren nach Anspruch 1, wobei die Zitrusschale einer Wärmebehandlung vor der Homogenisierungsbehandlung bei einer Temperatur von 50 °C bis 140 °C für einen Zeitraum von 1 Sekunde bis 3 Minuten unterzogen wird.

3. Verfahren nach Ansprüchen 1-2, wobei das Verfahren ferner einen Zerkleinerungs- oder Pulverisierungsschritt vor dem Desolventisieren und Trocknen umfasst.

4. Verfahren nach Ansprüchen 1-3, wobei das Verfahren ferner einen Zerkleinerungs- oder Pulverisierungsschritt nach dem Trocknen umfasst.

5. Verfahren nach Ansprüchen 1-4, wobei das Verfahren ferner das Aussetzen der Zitrusschale an ein Verarbeitungshilfsmittel umfasst, das ausgewählt ist aus der Gruppe bestehen aus Enzymen, Säuren, Basen, Hydrokolloiden, Gemüsefasern, Bleichmitteln und Kombinationen davon.

6. Verfahren nach Ansprüchen 1-5, wobei das Verfahren ferner das Hinzufügen eines Verarbeitungshilfsmittels umfasst, das ausgewählt ist aus der Gruppe bestehend aus Enzymen, Säuren, Basen, Hydrokolloiden, Gemüsefasern, Bleichmitteln und Kombinationen davon.

## Revendications

1. Un processus de préparation de fibres d'agrumes à partir de pelures d'agrumes usagées, le processus comprenant les étapes consistant à :
a. traiter des pelures d'agrumes usagées pour obtenir des pelures d'agrumes usagées homogénéisées ;
b. laver les pelures d'agrumes usagées homogénéisées avec un solvant organique pour obtenir des pelures d'agrumes usagées lavées au solvant organique ;
c. désolvanter et sécher les pelures d'agrumes usagées lavées au solvant organique ; et
d. en récupérer des fibres d'agrumes
et
dans lequel le traitement comprend l'homogénéisation sous pression en utilisant une pression allant de 50 bars à 1000 bars.

2. Un processus selon la revendication 1, dans lequel les pelures d'agrumes sont soumises à un traitement thermique avant le traitement d'homogénéisation à une température de 50° C à 140° C pendant une durée d'une seconde à 3 minutes.

3. Un processus selon les revendications 1 à 2, ledit processus comprenant en sus une étape de broyage ou pulvérisation avant la désolvantation et le séchage.

4. Un processus selon les revendications 1 à 3, d ledit processus comprenant en sus une étape de broyage ou pulvérisation après le séchage.

5. Un processus selon les revendications 1 à 4, ledit processus comprenant en sus le fait de soumettre lesdites pelures d'agrumes à un auxiliaire de traitement choisi dans le groupe consistant en des enzymes, des acides, des bases, des hydrocolloïdes, des fibres végétales, des agents de blanchiment et des combinaisons de ceux-ci.

6. Un processus selon les revendications 1 à 5, ledit processus comprenant en sus l'addition d'un auxiliaire de traitement choisi dans le groupe consistant en des enzymes, des acides, des bases, des hydrocolloïdes, des fibres végétales, des agents de blanchiment et des combinaisons de ceux-ci.
